# EUROPEAN PATENT SPECIFICATION

(11) **EP 2 672 828 B1**
(45) Date of publication and mention of the grant of the patent: **29.04.2015**
(21) Application number: 12701927.1
(22) Date of filing: 06.02.2012
(51) Int. Cl.: A01P 1/00, A01N 65/00, A01N 33/12, A61L 2/18, A61K 8/34, A61K 8/41, A61K 31/05, A61K 31/14

(54) **ANTIMICROBIAL COMPOSITION**
ANTIMIKROBIELLE ZUSAMMENSETZUNG
COMPOSITION ANTIMICROBIENNE

(30) Priority: 10.02.2011 IN MM03782011; 01.04.2011 EP 11160822
(43) Date of publication of application: 18.12.2013
(73) Proprietor: Unilever N.V., 3013 AL Rotterdam (NL); Unilever PLC, London, Greater London EC4Y 0DY (GB)
(72) Inventor: CHAKRABORTTY, Amit, Bangalore 560 066 (IN); DASGUPTA, Anindya, Bangalore 560 066 (IN); KHOKHAR, Jasmeet, Kaur, Bangalore 560 066 (IN); YEKHE, Ashish, Shrikant, Bangalore 560 066 (IN)
(74) Representative: van den Brom, Coenraad Richard
(86) International application number: PCT/EP2012/051940
(87) International publication number: WO 2012/107390

(56) References cited:
- WO-A1-01/41573
- WO-A1-03/089008
- WO-A1-2010/070664
- CN-C- 100 444 734
- DATABASE WPI Week 200936 Thomson Scientific, London, GB; AN 2009-G47450 XP002659132, & CN 101 380 447 A (ZHOU X) 11 March 2009 (2009-03-11)

## Description

### Filed of the invention

The invention relates to an antimicrobial composition. The invention more particularly relates to a composition comprising a combination of two materials that interact synergistically to provide virus kill in a short time.

### Background of the invention

Sanitizing/disinfecting is a very important aspect of ensuring healthy life in all populations of the world. Areas of focus for ensuring disinfection include personal uses like hand and body hygiene and hygiene of surfaces in homes, hotels, restaurants and other public places. Especially important in public hygiene is maintaining toilets, bathrooms, floors, kitchen tops and utensils in a sanitized state. Hygiene is especially important in places like hospitals where people come for cure of various diseases. While soap compositions (with or without natural or synthetic antimicrobial agents) have been used for personal hygiene; harsher antimicrobial actives like chlorine or alcohol containing compositions have been used for sanitizing hard surfaces. Alcohol containing hand sanitizers are also available.

Various types of microorganisms are known to cause different diseases. Bacteria, virus and protozoa are the three common types. There are compositions available for disinfection against each of these types of organisms. While very many antibacterial actives are available and widely used, killing of virus is more difficult and often requires harsher chemicals like chlorine or alcohol.

Therefore, there is a need for providing a composition that is relatively mild on the human skin while at the same time provides effective antiviral action. Antiviral action using such mild antiviral actives is known to be relatively slow and often taken several minutes, and in certain cases in order of hours. Thus there is a need for providing a mild antiviral composition that ensures antiviral kill in about 5 minutes or less, preferably lesser than 2 minutes.

Many materials of natural origin e.g. polyphenols, essential oils, etc are known to have some antiviral activity. Such types of compounds are found in many fruits, vegetables and other food items e.g. in tea. Further, many other synthetic compounds are used for providing various benefits in personal care compositions. However, very few compositions that provide fast anti-viral kill (in less than five minutes and in certain cases in less than one minute) have been reported in the area of personal care compositions, especially those which are substantially alcohol free. The present inventors have found that a combination of a cationic surfactant e.g. benzalkonium chloride and an extract of a rhizome viz. *Curcuma longa or curcuma aromatica* or its active ingredient viz. curcumin provide this fast antiviral action. This combination can be used to disinfect body surfaces and hard surfaces to substantially reduce incidence of both respiratory and gastrointestinal diseases.

US6248343 discloses an antimicrobial composition comprising: a) an antimicrobial selected from the group consisting of greater than 30% by volume alcohol, an effective amount of triclosan and mixtures thereof; and b) an effective amount of phenoxy ethanol, benzalkonium chloride or benzethonium chloride, and cocophosphatidyl-dimonium chloride; and c) an effective amount of naturally occurring plant or plant extract. Preferred naturally occurring plant or extract thereof is selected from the group consisting of curcuma longa, croccus sativus (saffron), alkanna tinctoria (henna root), hydrastis canadensis and mixtures thereof. The anti-microbial action in the above patent is largely due to effect of alcohol and therefore it is a challenge to have high and fast anti-viral action with low or minimal amount of alcohol.

GB2317339 discloses oral compositions comprising: a.) from about 0.001% to about 10% by weight, of a curcuminoid such as curcumin, curcumin derivatives, analogues, preparations of the plant Curcuma longa or other curcumin-containing plants, and mixtures thereof; b.) from about 0.005% to about. 0.35% of fluoride ion from a soluble fluoride ion source; c.) a safe and effective amount of at least one oral active such as an anticalculus agent, antimicrobial/antiplaque agent, antibacterial agent, and the like; d. from about 79% to about 98% of one or more orally-acceptable carrier materials; and e. additional antidental caries agents and anti-inflammatory agents. A large number of antimicrobial / antiplaque agents are listed of which benzalkonium chloride is one. This publication therefore does not clearly and unambiguously disclose a synergistic combination of a curcuma extract comprising curcumin and benzalkonium chloride for enhanced anti-viral action especially on skin or hard surface.

CN 101380447 (Zhou Xiaoping, 2009) discloses a foaming agent for curing gynaecological diseases comprising the following components: chitosan, mannan oligosaccharide, benzalkonium chloride, aloe oil, zedoary turmeric oil, triclosan, polyethylene nonylphenyl ether, coconut oil based alkyl sulphate among various other ingredients in a propellent (propane or butane based) composition. It is well established scientifically that Zedoary turmeric oil (from *Curcuma zedoaria*) contains practically no curcumin.

WO 2010/070664 discloses an aqueous ophthalmic composition, in the form of a gel or suspension for ocular diseases or disorders. The composition may comprise curcumin. Examples are provided that also comprise benzalkonium chloride at levels of 0.0097 to 0.0177%-wt.

WO 03/089008 discloses a precipitate, comprising at least an anionic polymeric component which is as such sluble in water and an amphiphilic ammonium-type component. The object of WO 03/089008 is to provide such precipitates for the slow release of medicaments. In an example, the precipitate comprises curcumin and an unspecified amount of benzalkonium chloride.

WO 01/41573 discloses antimicrobial compositions comprising at least 30 % alcohol and/or triclosan in combination with phenoxyethanol, benzalkonium or benzethonium chloride, cocophosphatidyl-dimonium chloride and plant extracts (preferably selected from curcuma longa, croccus sativus (saffron), alkanna tinctoria (henna root) and hydrastis canadensis (golden seal), for disinfecting skin.

CN 100 444 734 C relates to a virus atomizer, which uses oil of aromatic turmeric and Hypericum perforatum extracts. The invention is characterized in that it processes the volatile oil extracted from curcuma zedoaria rose and the flower-grass extract of Hypericum Peroratum is prepared into a solid, or liquid form to be filled into a micro atomizer to be sprayed.

It is thus an object of the present invention to provide for an antimicrobial composition that includes materials of natural origin (thus expected to be mild on skin) that is effective in substantial kill of virus.

It is another object of the present invention to provide for such effective yet mild antiviral compositions that ensures rapid kill of virus in less than about five minutes.

### Summary of the Invention

According to the first aspect of the present invention there is provided an antiviral composition comprising less than 1% C1 to C3 alcohol comprising:
(a) 0.001 to 5% by weight curcumin or extract of *Curcuma longa* or *Curcuma aromatica;*
(b) 0.02 to 5% by weight benzalkonium chloride, and
(c) a carrier.

According to another aspect of the invention there is provided a non-therapeutic method of disinfecting a surface comprising the steps of applying a composition as per the first aspect of the invention on to a desired surface.

According to yet another aspect of the invention there is provided non-therapeutic use of a composition comprising 0.001 to 5% by weight curcumin or *extract of Curcuma longa or Curcuma aromatica* and 0.02 to 5% by weight benzalkonium chloride for disinfecting a surface to be substantially free of virus.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarity the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as nodified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

The present inventors have found that a combination of a cationic surfactant e.g. benzalkonium chloride and an extract of a rhizome viz. *Curcuma longa or curcuma aromatica* or its active ingredient viz. curcumin provide this fast antiviral action. Therefore, the present invention relates to a composition comprising less than 1% C1 to C3 alcohol comprising
(a) 0.001 to 5% by weight curcumin or extract of *Curcuma longa* or *Curcuma aromatica;*
(b) 0.02 to 5% by weight benzalkonium chloride, and
(c) a carrier.

The antiviral composition comprises curcumin or extract of *Curcuma longa or curcuma aromatica,* benzalkonium chloride and a carrier. Various components of the antiviral composition are described below. The compositions of the present invention are preferred for non-therapeutic use, and more particularly preferred for use in cleaning surfaces of human body including skin or for hard surface cleaning applications. By an anti-viral composition is meant a composition that provides substantial reduction in viral count on treatment with said composition.

The *Curcuma* extract preferably comprises 0.1 to 5 , more preferably 0.1 to 1 percent of the active curcumin. Else, the compound curcumin may be incorporated in the composition in pure form either extracted from natural sources or prepared synthetically. *Curcuma* extract or curcumin is preferably present in 0.05 to 1, more preferably 0.1 to 1 by weight of the composition.

Curcumin has the structure given below.

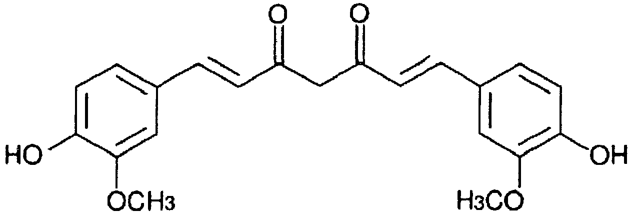

The composition of the invention comprises 0.02 to 5% benzalkonium chloride. Benzalkonium chloride is a cationic surfactant having the structure given below:

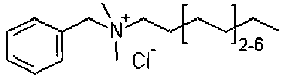

Benzalkonium chloride is preferably present in 0.05 to 3%, by weight of the composition.

The composition of the invention comprises a carrier. The carrier is preferably selected from water, oil, solvent, inorganic particulate material, starch and mixtures thereof. The carrier is preferably from 0.1 to 99% by weight of the composition. The antimicrobial composition may be in form of a solid, liquid, gel, paste or soft solid and the carrier may be selected by a person skilled in the art depending on the format of the antimicrobial composition.

The examples of inorganic particulate materials include clay, talc, calcite, dolomite, silica, and aluminosilicate. The examples of oils include mineral oils, vegetable oils, and petroleum-derived oils and waxes. The examples of solvents include alcohols, ethers and acetone.

The starch may be natural starch obtained from food grains or may be a modified starch.

Particularly preferred carriers are water or oil/solvent, more preferred a carrier being a mixture of water and oil. In most of the envisaged applications like personal washing, antiseptic liquid, or for hard surface cleaning, the composition of the invention may be formulated in an aqueous base (water being carrier) e.g. products in gel format or in purely oil/solvent base e.g. products in anhydrous stick form or propellant containing products. However, most preferred product formats have an emulsion base (water and oil being the carrier) e.g. soap products in liquid, solid, lotion or semisolid form for hand wash, face wash, body wash, or shaving applications; or products for hard surface cleaning in bars or liquids form.

The composition preferably comprises 1 to 80% surfactant. In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

A particularly preferred surfactant is soap. By soap is meant a salt of fatty acid. Soap is a suitable surfactant for personal washing applications of the composition of the invention. The soap is preferably C8-C24 soap, more preferably C10-C20 soap and most preferably C12-C16 soap where the carbon chain length refers to the carbon chain length of the fatty acid used to make the soap. The soap may or may not have one or more carbon-carbon double bond or triple bond. The cation of the soap can be alkali metal, alkaline earth metal or ammonium. Preferably, the cation of the soap is selected from sodium, potassium or ammonium. More preferably the cation of the soap is sodium or potassium.

The composition of the invention can be formulated as a liquid personal wash composition comprising 0.001 to 5% by weight curcumin or extract of *Curcuma longa or Curcuma aromatica;* 0.02 to 5% by weight benzalkonium chloride, and a carrier comprising 10 to 99% by weight water; and 0.1 to 30% by weight anionic surfactant. The anionic surfactant is preferably soap. By a personal wash composition is meant a composition to wash the external surface of mammals preferably human beings e.g. external surfaces like hair, face, body, hands and the axilia. It is particularly preferred that the liquid personal wash composition is used for hand wash applications.

The composition of the invention can also be formulated as a solid personal wash composition comprising 0.001 to 5 weight% curcumin or extract of *Curcuma longa or Curcuma aromatica;* 0.02 to 5% by weight benzalkonium chloride, and a carrier comprising of 5 to 40% by weight water; and 30 to 90% by weight anionic surfactant. The anionic surfactant is preferably soap. The solid personal wash composition is preferably a soap bar.

A composition of the invention is especially useful as a liquid antiseptic composition. By an antiseptic composition is meant a composition that may be used as such or after dilution with water for first aid treatment of cuts on skin and other keratinous substrates of the body, for disinfecting surfaces which are believed to be high in germ count e.g. nappies, hospital linen, undergarments, and hardsurfaces like those in the kitchen, bathrooms and toilets. Liquid antiseptic composition is a class of products where the composition is predominantly water or a mix of water and solvents with a small amount of disinfectants which may be synthetically prepared or of natural origin. Providing such a composition in a transparent or translucent form is highly appealing to the consumer. In order to facilitate ease of packing and transportation, these products are often manufactured at much higher concentration of antimicrobial actives than are actually required in use. The consumers are instructed to dilute the product with water before actual use. In addition to the providing the consumers with a transparent or translucent composition when packed it is important to provide the consumer with a visual cue that the product has been sufficiently diluted for actual use. Such a cue could be change of the formulation from a transparent/ translucent form to the formation of a turbid suspension or cloudiness on dilution to the desired level.

The invention thus provides for a liquid antiseptic composition comprising 0.001 to 5% by weight curcumin or *extract of Curcuma longa or curcuma aromatica;* 0.02 to 5% by weight benzalkonium chloride, and a carrier comprising water at 10 to 99% by weight of the composition.

The liquid antiseptic composition of the invention is usually to be used for disinfecting a surface by first diluting the composition in water. The preferred weight ratio of composition: water for the dilution step is in the range of 1:1 to 1:200, more preferably 1:5 to 1:50, further more preferably 1:10 to 1:50, optimally 1:15 to 1:30 and ideally about 1:20.

The composition of the invention is especially useful in hard surface cleaning applications. In such applications preferred surfactants are nonionic surfactants, such as C8-C22, preferably C8-C16 fatty alcohol ethoxylates, comprising between 1 and 8 ethylene oxide groups when the product is in the liquid form. When the product is in the solid form for hard surface cleaning applications surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16. Suitable surfactant concentrations in liquid forms of hard surface cleaning application are generally from about from 0.1 to 10%, preferably 0.5 to 10%, more preferably from 1 to 5 % by weight of the composition. In solid compositions, surfactant is preferably present in 5 to 40%, preferably from 10 to 30% by weight of the composition. By hard-surface cleaning is meant a compostion for cleaning hard surfaces like utensils and cutlery used in the kitchen,

The invention thus provides for a solid hard surface cleaning composition comprising 0.001 to 5% by weight curcumin or extract of *Curcuma longa or Curcuma aromatica;* 0.02 to 5% by weight benzalkonium chloride and a carrier comprising 5 to 40% by weight water; and 10 to 30% by weight anionic surfactant.

The invention also provides for a liquid hard surface cleaning composition comprising 0.001 to 5% by weight curcumin or extract of *Curcuma longa or Curcuma aromatica;* 0.02 to 5% by weight benzalkonium chloride and a carrier comprising 10 to 99% by weight water; and 0.1 to 10% by weight anionic or non-ionic surfactant.

The anionic surfactant in hard surface cleaning applications is preferably selected from primary alkyl sulphate, secondary alkyl sulphonates, alkyl benzene sulphonates, or ethoxylated alkyl sulphates.

The composition of the invention is also preferably formulated as a hand sanitizer. Hand sanitisers are those class of products that are sold as thickened liquids (or gels) in small bottles. People who do not have access to soap and/or water in public places and desire to consume some food or drink using their hands while ensuring that their hands are germ free, use such products. They squeeze a drop or two of the thickened liquid on their hands and rub them together. The liquid kills the germs and thereafter evaporates leaving the hands dry and substantially germ free. One of the most commonly used active in conventional hand sanitizers is a C1 to C3 alcohol e.g. ethanol. The present invention is especially advantageous since it is directed at minimizing use of alcohol, while being equally if not more effective in killing virus. The present invention thus provides for a composition comprising 0.001 to 5% by weight curcumin or extract of *Curcuma longa or Curcuma aromatica;* 0.02 to 5% by weight benzalkonium chloride, and a carrier comprising 0.03% to 5% thickening polymer and 10 to 99% water.

The composition of the invention additionally preferably comprises an antibacterial active. Antibacterial actives of natural origin are especially preferred. Essential oils are a class of antibacterial actives useful in including in the composition of the present invention, to get broad antimicrobial activity. They are especially useful for including in the hand sanitizer aspect of the present invention. Antibacterial essential oil is preferably selected from amyl salicylate, carvacrol, cymene, e.g. p-cymene, dihydroeugenol, eugenol, hexyl eugenol, hexyl salicylate, isoeugenol, methyl eugenol, methyl isoeugenol, methyl salicylate, tert butyl cresol, thymol, vanillin, cedrene, cineole, citral (including geranial and neral), citronellal, citronellol, eucalyptol (also known as 1,8 cineole) paradihydrolinalool, dihydromyrcenol (DH myrcenol), farnesol, geraniol, hexyl cinnamaldehyde, hydroxycitronallol, hydroxycitronellal, isocitral, limonene, preferably d-limonene, linalool, longifolene, menthol, nerol, nerolidiol, pinene, e.g. α-pinene, phellendrene, terpinene, e.g. α-terpinene and γ-terpinene, terpineol, e.g. γ-terpineol and terpin-4-ol, and tetrahydromyrcenol (THM). Of these the antibacterial essential oil especially preferred are terpineol, thymol, eugenol or mixtures thereof.

The inventors have determined to their utter surprise that a combination of extract of *Curcuma* longa or Curcuma aromatic or curcumin along with benzalkonium chloride at selective concentrations provides a synergistic antiviral action which is especially important in a wash off processes where the contact time of the antimicrobial actives with the surface is low, i.e. of the order of less than 5 minutes, preferably less than 2 minutes. Fortuitously such wash off processes include a surfactant for the cleaning action.

The composition may further comprise various additional ingredients known to a person skilled in the art. Such additional ingredients include but are not limited to: perfumes, pigments, preservative, emollients, sunscreens, emulsifiers, gelling agents, or thickening agents.

The compositions of the present invention may be in form of a solid, a liquid, a gel or a paste. The compositions of the present invention are useful for cleansing and care, in particular for skin cleansing and skin care. It is envisaged that the composition can be used as a leave-on product or a wash-off product, preferably a wash-off product. The composition of the present invention can also be used for cleansing and care of hard surfaces such as glass, metal, plastic and the like.

The present invention is especially useful since it envisages it to be substantially free of low molecular weight alcohol viz. C1 to C3 alcohols e.g. ethanol or isopropyl alcohol. By the term substantially free of low molecular weight alcohols it is meant that the alcohol is present in an amount which does not significantly affect the microbial kill. Preferably C1 to C3 alcohols is present in less than 1%, preferably less than 0.5%, further more preferably less than 0.1% and most preferably absent from the composition of the invention.

The invention also provides for a method of disinfecting a surface comprising the steps of applying a composition of the invention on to a desired surface. Desired surfaces are external skin surface of a human body or any inanimate hard surface. After the step of applying the composition on to the desired surface, it is preferably followed by a step of rinsing or wiping the surface with a suitable solvent or wipe, a desired time period after applying the composition. This step of rinsing or wiping the surface is preferably carried out less than 5 minutes after the step of applying the composition on the surface.

According to yet another aspect of the present invention there is provided use of a composition comprising 0.001 to 5% by weight curcumin or extract of *Curcuma* longa or Curcuma aromatic and 0.02 to 5% by weight benzalkonium chloride for disinfecting a surface to be substantially free of virus. The virus to be disinfected includes non-enveloped (Feline Calicivirus or MS2 bacteriophage) or enveloped virus (Respiratory Syncitial Virus). The use is preferably for non-therapeutic purposes.

The invention will now be illustrated with the help of the following non-limiting examples.

### Examples

The virucidal activity of various test solutions were measured using the following protocol.

### Determination of Virucidal Activity

A standard EST protocol (EN14476) was followed for the evaluation of activity of antiviral actives in suspension test. Antiviral activity of actives was tested in clean conditions.

Clean conditions: To create clean conditions, 0.3 g of bovine albumin fraction V was dissolved in 100 ml of water and sterilised by passing the solution through a 0.2 µm filter).

Preparation of test mixture: To prepare the test mixture, 1 ml of interfering substance (Bovine Albumin) was pipetted into a suitable container, followed by addition of 1 ml of test virus suspension. To this, 8 ml of product test solution was added. At the end of chosen contact time period, 100µl of the test mixture was added to resin column. Centrifugation of microspin column (735 xg, 2 min). The elutant was diluted upto 10⁻⁶ in ice cold virus growth medium. The infectivity of each dilution was measured by TCID₅₀ using the Spaerman-Karber formula.

Titration of Virus: Monolayers of cells were grown in 96-well tissue-culture plates (Costar) at 37°C in an atmosphere of 95% air and 5% carbon dioxide (CO₂). Tenfold dilutions of virus suspension were prepared in virus growth medium. One hundred microlitres of each dilution was added to five replicate wells in 96-well tissue-culture plates. Cell controls were included on each plate. Plates were incubated at 37°C in an atmosphere of 95% air/ 5% CO₂. Cultures were observed daily for viral cytopathic effect for a period of seven days after which they were discarded. The virus titre was calculated by TCID₅₀ (Tissue culture infective dose) using the Spaerman-Karber formula.

The formula is:
Negative logarithm of the 50 % end point = Negative logarithm of the highest virus concentration used - [(Sum of % affected at each dilution / 100 - 0,5) x (lg of dilutions)]

### Examples 1 -4: Effect of various compositions on viral kill of FCV:

Various compositions as shown in Table - 1 were prepared and their virucidal activity was measured using the procedure given earlier. The data on amount of virus remaining as measured using log (TCID₅₀)/0.1ml is also given in Table -1. The antiviral actives were contacted with the virus for a period of time of five minutes.

**Table - 1**

| Examples | Test Sample | Log TCID₅₀/0.1 ml |
|---|---|---|
| 1 | FCV Control (Feline Calcivirus) | 4.50 |
| 2 | 0.1% BKC* | 3.25 |
| 3 | 0.25% Curcumin | 3.40 |
| 4 | 0.1% BKC and 0.25% Curcumin | 1.30 |

| | | |
|---|---|---|
| * BKC refers to benzalkonium chloride | | |

The data in Table - 1 indicates that a combination of BKC and Curcumin provides for vastly superior antiviral activity against non-enveloped virus like FCV.

### Examples 5 -8: Effect of various compositions on virus kill of RSV:

Various compositions as shown in Table - 2 were prepared and their virucidal activity was measured using the procedure given earlier. The data on amount of virus remaining as measured using log (TCID₅₀)/0.1ml is also given in Table -2. The antiviral actives were contacted with the virus for a period of time of five minutes.

**Table - 2**

| Examples | Test Sample | Log TCID₅₀/0.1 ml |
|---|---|---|
| 5 | RSV Control (Respiratory syncitial virus) | 5.00 |
| 6 | 0.1 % BKC | 2.50 |
| 7 | 0.25% Curcumin | 4.65 |
| 8 | 0.1% BKC and 0.25% Curcumin | 1.50 |

The data in Table - 2 indicates that a combination of BKC and Curcumin provides for vastly superior antiviral activity against an enveloped virus like RSV.

### Examples 9 -12: Effect of various compositions on virus kill of MS-2 bacteriophage:

Various compositions as shown in Table - 3 were prepared and their virucidal activity was measured using the procedure given earlier. The data on amount of virus remaining as measured using log (TCID₅₀)/0.1ml is also given in Table -3. The antiviral actives were contacted with the virus for a period of time of five minutes.

**Table - 3**

| Examples | Test Sample | Log TCID50/0.1 ml |
|---|---|---|
| 9 | MS2 Phage | 6.36 |
| 10 | 0.105% BKC | 1.94 |
| 11 | 0.105% Curcuma extract | 6.22 |
| 12 | 0.1% BKC and 0.005% *Curcuma* extract | 1.36 |

The data in Table - 1 indicates that a combination of BKC and *Curcuma* extract provides for synergistic antiviral activity against MS-2 phage.

### Examples 13 -16: Effect of various compositions on kill of FCV:

Various compositions as shown in Table - 4 were prepared and their virucidal activity was measured using the procedure given earlier. The data on amount of virus remaining as measured using log (TCID₅₀)/0.1ml is also given in Table -4. The antiviral actives were contacted with the virus for a period of time of five minutes.

**Table - 4**

| Examples | Test Sample | Log TCID50/0.1 ml |
|---|---|---|
| 13 | FCV Control | 5.50 |
| 14 | 0.105% BKC | 3.10 |
| 15 | 0.105% Curcuma extract | 4.50 |
| 16 | 0.1% BKC and 0.005% *Curcuma* extract | 2.50 |

The data in Table - 4 indicates that a combination of BKC and *Curcuma* extract provides for synergistic antiviral activity against non-enveloped virus like FCV.

### Examples 17 -20: Effect of various compositions on kill of RSV:

Various compositions as shown in Table - 5 were prepared and their virucidal activity was measured using the procedure given earlier. The data on amount of virus remaining as measured using log (TCID₅₀)/0.1ml is also given in Table -5. The antiviral actives were contacted with the virus for a period of time of five minutes.

**Table - 5**

| Examples | Test Sample | Log TCID₅₀/0.1 ml |
|---|---|---|
| 17 | RSV Control | 4.70 |
| 18 | 0.105% BKC | 3.90 |
| 19 | 0.105o Curcuma extract | 3.90 |
| 20 | 0.1% BKC and 0.005% *Curcuma* extract | 3.10 |

The data in Table - 5 indicates that a combination of BKC and *Curcuma* extract provides for synergistic antiviral activity against enveloped virus like RSV.

## Claims

1. An antiviral composition comprising less than 1% C1 to C3 alcohol comprising:
(a) 0.001 to 5% by weight curcumin or extract of *Curcuma longs* or *Curcuma aromatica;*
(b) 0.05 to 5% by weight benzalkonium chloride, and
(c) a carrier.

2. A composition as claimed in claim 1, wherein the composition is a liquid antiseptic composition, and wherein said carrier is water at 10 to 99% by weight of the composition.

3. A composition as claimed in claim 1, wherein the composition is a liquid personal wash composition, and wherein said carrier comprises 10 to 99% by weight water; and 0.1 to 30% by weight anionic surfactant.

4. A composition as claimed in claim 1, wherein the composition is a solid personal wash composition, and wherein said carrier comprises 5 to 40% by weight water; and 30 to 90% by weight anionic surfactant.

5. A' composition as claimed in claim 3 or 4 wherein said surfactant is soap.

6. A composition as claimed in claim 1, wherein the composition is a solid hard surface cleaning composition, and wherein said carrier comprises 5 to 40% by weight water; and 10 to 30% by weight anionic surfactant.

7. A composition as claimed in claim 1, wherein the composition is a liquid hard surface cleansing composition, and wherein said carrier comprises 10 to 99% by weight water; and 0.1 to 10% by weight anionic or non-ionic surfactant.

8. A composition as claimed in claim 6 or 7 wherein said anionic surfactant is selected from primary alkyl sulphate, secondary alkyl sulphonates, alkyl benzene sulphonates, or ethoxylated alkyl sulphates.

9. A composition as claimed in claim 1, wherein the composition is an antimicrobial composition comprising an antibacterial essential oil selected from amyl salicylate, carvacrol, cymene, dihydroeugenol, eugenol, hexyl eugenol, hexyl salicylate, isoeugenol, methyl eugenol, methyl isoeugenol, methyl salicylate, tert butyl cresol, thymol, vanillin, cedrene, cineole, citral, citronellal, citronellol, eucalyptol (paradihydrolinalool, dihydromyrcenol, farnesol, geraniol, hexyl cinnamaldehyde, hydroxycitronallol, hydroxycitronellal, isocitral, limonene, linalool, longifolene, menthol, nerol, nerolidiol, pinene, phellendrene, terpinene, terpineol, and tetrahydromyrcenol.

10. A composition as claimed in claim 9 wherein said antibacterial essential oil is selected from terpineol, thymol, euganol or mixtures thereof.

11. A composition as claimed in claim 1, wherein the composition is a hand sanitizer composition, and wherein said carrier comprises 0.03% to 5% thickening polymer and 10 to 99% water.

12. A non-therapeutic method of disinfecting a surface comprising the steps of applying a composition as claimed in any one of the preceding claims on to a desired surface.

13. A method as claimed in claim 12 comprising the step of rinsing or wiping the surface with a suitable solvent or wipe, a desired time period after applying the composition.

14. A non-therapeutic method of disinfecting a surface using a composition of claim 1 comprising the step of diluting the composition with water in a weight ratio of 1: 1 to 1:200 before applying the composition on the desired surface.

15. Non-therapeutic use of a composition comprising 0.001 to 5% by weight Curcumin or extract of *Curcuma longa or Curcuma aromatica* and 0.02 to 5% by weight benzalkonium chloride for disinfecting a surface to be substantially free of virus.

## Patentansprüche

1. Antivirale Zusammensetzung, die weniger als 1 % C₁-C₃-Alkohol aufweist, die Folgendes aufweist:
(a) 0,001 bis 5 Gew.-% Curcumin oder Extrakt von Curcuma longa oder Curcuma aromatica;
(b) 0,05 bis 5 Gew.-% Benzalkoniumchlorid und
(c) einen Träger.

2. Zusammensetzung nach Anspruch 1,
wobei die Zusammensetzung eine flüssige antiseptische Zusammensetzung ist und wobei der Träger Wasser mit 10 bis 99 Gew.-% der Zusammensetzung ist.

3. Zusammensetzung nach Anspruch 1,
wobei die Zusammensetzung eine flüssige Körperwäschezusammensetzung ist und wobei der Träger 10 bis 99 Gew.-% Wasser und 0,1 bis 30 Gew.-% anionisches Tensid aufweist.

4. Zusammensetzung nach Anspruch 1,
wobei die Zusammensetzung eine feste Körperwäschezusammensetzung ist und wobei der Träger 5 bis 40 Gew.-% Wasser und 30 bis 90 Gew.-% anionisches Tensid aufweist.

5. Zusammensetzung nach Anspruch 3 oder 4,
wobei das Tensid Seife ist.

6. Zusammensetzung nach Anspruch 1,
wobei die Zusammensetzung eine feste Reinigungszusammensetzung für harte Oberflächen ist und wobei der Träger 5 bis 40 Gew.-% Wasser und 10 bis 30 Gew.-% anionisches Tensid aufweist.

7. Zusammensetzung nach Anspruch 1,
wobei die Zusammensetzung eine flüssige Reinigungszusammensetzung für harte Oberflächen ist und wobei der Träger 10 bis 99 Gew.-% Wasser und 0,1 bis 10 Gew.-% anionisches oder nichtionisches Tensid aufweist.

8. Zusammensetzung nach Anspruch 6 oder 7,
wobei das anionische Tensid aus primärem Alkylsulfat, sekundären Alkylsulfonaten, Alkylbenzolsulfonaten oder ethoxylierten Alkylsulfaten ausgewählt ist.

9. Zusammensetzung nach Anspruch 1,
wobei die Zusammensetzung eine antimikrobielle Zusammensetzung ist, die ein antibakterielles essenzielles Öl aufweist, das ausgewählt ist aus Amylsalicylat, Carvacrol, Cymol, Dihydroeugenol, Eugenol, Hexyleugenol, Hexylsalicylat, Isoeugenol, Methyleugenol, Methylisoeugenol, Methylsalicylat, tert.-Butylcresol, Thymol, Vanillin, Cedren, Cineol, Citral, Citronellal, Citronellol, Eukalyptol (Paradihydrolinalool), Dihydromyrcenol, Farnesol, Geraniol, Hexylzimtaldehyd, Hydroxycitronallol, Hydroxycitronellal, Isocitral, Limonen, Linalool, Longifolen, Menthol, Nerol, Nerolidiol, Pinen, Phellendren, Terpinen, Terpineol und Tetrahydromyrcenol.

10. Zusammensetzung nach Anspruch 9,
wobei das antibakterielle essenzielle Öl aus Terpineol, Thymol, Eugenol oder Gemischen davon ausgewählt ist.

11. Zusammensetzung nach Anspruch 1,
wobei die Zusammensetzung eine Handdesinfektionszusammensetzung ist und wobei der Träger 0,03 bis 5 % verdickendes Polymer und 10 bis 99 % Wasser aufweist.

12. Nichttherapeutisches Verfahren zum Desinfizieren einer Oberfläche,
das die Schritte des Auftragens einer Zusammensetzung nach einem der vorstehenden Ansprüche auf eine gewünschte Oberfläche aufweist.

13. Verfahren nach Anspruch 12,
das den Schritt des Spülens oder Abwischens der Oberfläche mit einem geeigneten Lösungsmittel oder Tuch nach einem gewünschten Zeitraum nach dem Auftragen der Zusammensetzung aufweist.

14. Nichttherapeutisches Verfahren zum Desinfizieren einer Oberfläche unter Verwendung einer Zusammensetzung nach Anspruch 1,
das den Schritt des Verdünnens der Zusammensetzung mit Wasser in einem Gewichtsverhältnis von 1:1 bis 1:200 vor dem Auftragen der Zusammensetzung auf die gewünschte Oberfläche aufweist.

15. Nichttherapeutische Verwendung einer Zusammensetzung, die 0,001 bis 5 Gew.-% Curcumin oder Extrakt von Curcuma longa oder Curcuma aromatica und 0,02 bis 5 Gew.-% Benzalkoniumchlorid aufweist, zum Desinfizieren einer Oberfläche, damit sie im Wesentlichen virusfrei ist.

## Revendications

1. Composition antivirale comprenant moins de 1 % de C1 à C3 alcool comprenant :
(a) 0,001 à 5 % en poids de curcumine ou d'extrait de *Curcuma longa* ou *Curcuma aromatica ;*
(b) 0,05 à 5 % en poids de chlorure de benzalkonium, et
(c) un vecteur.

2. Composition selon la revendication 1, où la composition est une composition antiseptique liquide et où ledit vecteur est l'eau à raison de 10 à 99 % en poids de la composition.

3. Composition selon la revendication 1, où la composition est une composition de lavage personnel liquide, et où ledit vecteur comprend 10 à 99 % en poids d'eau ; et 0,1 à 30 % en poids de tensioactif anionique.

4. Composition selon la revendication 1, où la composition est une composition de lavage personnel solide, et où ledit vecteur comprend 5 à 40 % en poids d'eau ; et 30 à 90 % en poids de tensioactif anionique.

5. Composition selon la revendication 3 ou 4 où ledit tensioactif est un savon.

6. Composition selon la revendication 1, où la composition est une composition de nettoyage de surface dure solide, et où ledit vecteur comprend 5 à 40 % en poids d'eau ; et 10 à 30 % en poids de tensioactif anionique.

7. Composition selon la revendication 1, où la composition est une composition de nettoyage de surface dure liquide, et où ledit vecteur comprend 10 à 99 % en poids d'eau ; et 0,1 à 10 % en poids de tensioactif anionique ou non ionique.

8. Composition selon la revendication 6 ou 7 où ledit tensioactif anionique est choisi parmi un alkylsulfate primaire, les alkylsulfonates secondaires, les alkylbenzènesulfonates et les alkylsulfates éthoxylés.

9. Composition selon la revendication 1, où la composition est une composition antimicrobienne comprenant une huile essentielle anti-bactérienne choisie parmi le salicylate d'amyle, le carvacrol, le cymène, le dihydroeugénol, l'eugénol, l'hexyleugénol, le salicylate d'hexyle, l'isoeugénol, le méthyleugénol, le méthylisoeugénol, le salicylate de méthyle, le tert-butylcrésol, le thymol, la vanilline, le cédrène, le cinéole, le citral, le citronellal, le citronellol, l'eucalyptol (le paradihydrolinalool, le dihydromyrcénol, le farnésol, le géraniol, l'hexylcinnamaldéhyde, l'hydroxycitronallol, l'hydroxycitronellal, l'isocitral, le limonène, le linalool, le longifolène, le menthol, le nérol, le nérolidiol, le pinène, le phellendrène, le terpinène, le terpinéol et le tétrahydromyrcénol.

10. Composition selon la revendication 9 où ladite huile essentielle antibactérienne est choisie parmi le terpinéol, le thymol, l'eugénol ou leurs mélanges.

11. Composition selon la revendication 1, où la composition est une composition pour l'hygiène des mains, et où ledit vecteur comprend 0,03 % à 5 % de polymère épaississant et 10 à 99 % d'eau.

12. Procédé non thérapeutique de désinfection d'une surface comprenant les étapes d'application d'une composition selon l'une quelconque des revendications précédentes sur une surface souhaitée.

13. Procédé selon la revendication 12 comprenant l'étape de rinçage ou d'essuyage de la surface avec un solvant ou un linge approprié, une durée souhaitée après l'application de la composition.

14. Procédé non thérapeutique de désinfection d'une surface au moyen d'une composition selon la revendication 1 comprenant l'étape de dilution de la composition avec de l'eau dans un rapport en poids de 1:1 à 1:200 avant l'application de la composition sur la surface souhaitée.

15. Utilisation non thérapeutique d'une composition comprenant 0,001 à 5 % en poids de curcumine ou d'extrait de *Curcuma longa* ou *Curcuma aromatica* et 0,02 à 5 % en poids de chlorure de benzalkonium pour désinfecter une surface pour qu'elle soit sensiblement dépourvue de virus.
